# EUROPEAN PATENT SPECIFICATION

(11) **EP 1 117 403 B1**
(45) Date of publication and mention of the grant of the patent: **10.12.2003**
(21) Application number: 99948905.7
(22) Date of filing: 30.09.1999
(51) Int. Cl.: A61K 31/44, A61P 25/24, A61P 29/00

(54) **MGLUR5 ANTAGONISTS FOR THE TREATMENT OF PAIN AND ANXIETY**
MGLUR5 ANTAGONISTEN ZUR BEHANDLUNG VON SCHMERZEN UND ANGSTZUSTÄNDEN
ANTAGONISTES DU MGLUR5 POUR LE TRAITEMENT DE LA DOULEUR ET DE L'ANGOISSE

(30) Priority: 02.10.1998 GB 9821503; 23.12.1998 US 220813
(43) Date of publication of application: 25.07.2001
(73) Proprietor: Novartis AG, 4056 Basel (CH); Novartis Pharma GmbH, 1230 Wien (AT); Sibia Neurosciences, Inc., La Jolla, CA 92037-4641 (US)
(72) Inventor: ALLGEIER, Hans, D-79541 Lörrach (DE); COSFORD, Nicholas, David, San Diego, CA 92122 (US); FLOR, Peter, Josef, 4053 Basel (CH); GASPARINI, Fabrizio, CH-4415 Lausen (CH); GENTSCH, Conrad, CH-4102 Binningen (CH); HESS, Stephen, D., San Diego, CA 92129 (US); JOHNSON, Edwin, Carl, San Diego, CA 92129 (US); KUHN, Rainer, D-79540 Lörrach (DE); TRICKLEBANK, Mark, Teddington TW 11 9PP (GB); URBAN, Laszlo, London NI4 7ET (GB); VARNEY, Mark, Andrew, San Diego, CA 92129 (US); VELI ELEBI, Gönül, San Diego, CA 92130 (US); WALKER, Katharine, London SW11 1HE (GB)
(74) Representative: Grubb, Philip William
(86) International application number: EP9907239
(87) International publication number: WO00020001

(56) References cited:
- WO-A-99/02497
- MORI M ET AL: "THE NEMATICIDAL ACTIVITY OF ACETYLENE COMPOUNDS" AGRICULTURAL AND BIOLOGICAL CHEMISTRY,JP,JAPAN SOC. FOR BIOSCIENCE, BIOTECHNOLOGY AND AGROCHEM. TOKYO, vol. 46, no. 1, page 309-311 XP000645051 ISSN: 0002-1369
- FUNDYTUS M E ET AL: "In vivo antinociceptive activity of anti-rat mGluR1 and mGluR5 antibodies in rats." NEUROREPORT, (1998 MAR 9) 9 (4) 731-5. , XP002128754
- MORTIN-TOTH, STEVE (1) ET AL: "Unilaterally increased expression of mGluR5 in dorsal horn neurons following knee joint inflammation." SOCIETY FOR NEUROSCIENCE ABSTRACTS, (1997) VOL. 23, NO. 1-2, PP. 1809. MEETING INFO.: 27TH ANNUAL MEETING OF THE SOCIETY FOR NEUROSCIENCE NEW ORLEANS, LOUISIANA, USA OCTOBER 25-30, 1997 , XP002128755
- KNÖPFEL T. ET AL: "Metabotropic Glutamate Receptors: Novel targets for drug development" J. MED. CHEM., vol. 38, no. 9, 1995, pages 1417-1426, XP002128756 cited in the application
- VARNEY M A ET AL: "SIB-1757 and SIB-1893: selective, noncompetitive antagonists of metabotropic glutamate receptor type 5." JOURNAL OF PHARMACOLOGY AND EXPERIMENTAL THERAPEUTICS, (1999 JUL) 290 (1) 170-81. , XP002128757
- BOWES, M. (1) ET AL: "Anti- hyperalgesic effects of the novel metabotropic glutamate receptor 5 antagonist, 2-methyl-6- (phenylethynyl)-pyridine, in rat models of inflammatory pain." BRITISH JOURNAL OF PHARMACOLOGY, (MARCH, 1999) VOL. 126, NO. PROC. SUPPL. PP. 250P. MEETING INFO.: MEETING OF THE BRITISH PHARMACOLOGICAL SOCIETY BRIGHTON, LONDON, ENGLAND JANUARY 6-8, 1999 BRITISH PHARMACOLOGICAL SOCIETY. , XP002128758
- VARNEY, M. A. (1) ET AL: "Characterization of SIB-1757 and SIB-1893: Highly selective antagonists a metabotropic glutamate receptor subtype 5." BRITISH JOURNAL OF PHARMACOLOGY, (MARCH, 1999) VOL. 126, NO. PROC. SUPPL. PP. 248P. MEETING INFO.: MEETING OF THE BRITISH PHARMACOLOGICAL SOCIETY BRIGHTON, LONDON, ENGLAND JANUARY 6-8, 1999 BRITISH PHARMACOLOGICAL SOCIETY. , XP002128759
- GASPARINI, F. (1) ET AL: "2-methyl-6-(phenylethynyl)-pyridine (MPEP): A novel potent, subtype-selective and systemically active antagonist at metabotropic glutamate receptor subtype 5." BRITISH JOURNAL OF PHARMACOLOGY, (MARCH, 1999) VOL. 126, NO. PROC. SUPPL. PP. 249P. MEETING INFO.: MEETING OF THE BRITISH PHARMACOLOGICAL SOCIETY BRIGHTON, LONDON, ENGLAND JANUARY 6-8, 1999 BRITISH PHARMACOLOGICAL SOCIETY. , XP002128760

## Description

The present invention relates to new pharmaceutical uses of compounds having antagonistic activity at metabotropic glutamate receptors (mGluRs).

Glutamate is the principal excitatory transmitter in the central nervous system acting through ionotropic glutamate receptors. It also plays a major role in activating modulatory pathways through the mGluRs.

Based on their amino acid sequence homology, agonist pharmacology and coupling to transduction mechanisms, the 8 presently known mGluR sub-types are classified into three groups. Group I receptors (mGluR1 and mGluR5) have been shown to be coupled to stimulation of phospholipase C resulting in phosphoinositide hydrolysis and elevation of intracellular Ca⁺⁺ levels, and, in some expression systems, to couple to modulation of ion channels, such as K⁺ channels, Ca⁺⁺ channels, non-selective cation channels or NMDA receptors. Group II receptors (mGluR2 and mGluR3) and Group III receptors (mGluRs 4, 6, 7 and 8) are negatively coupled to adenylylcyclase and have been shown to couple to inhibition of cAMP formation when heterologously expressed in mammalian cells, and to G-protein-activated inward rectifying potassium channels in Xenopus oocytes and in unipolar brush cells in the cerebellum. Besides mGluR6, which is essentially only expressed in the retina, the mGluRs are felt to be widely expressed throughout the central nervous system (CNS).

Said mGluRs have been implicated as potentially important therapeutic targets for a number of neurological and psychiatric disorders largely based on studies with compounds not discriminating between mGluR subtypes (for review see Knopfel et al., J. Med. Chem. 38, 1417-26, 1995; Conn and Pin, Annu. Rev. Pharmacol. Toxicol. 37, 205-37, 1997). Particularly, for group I mGluR, the elucidation of the role of the individual receptor subtypes has been significantly hampered by the lack of potent, systemically active, subtype-selective compounds.

According to the present invention it has unexpectedly been found that selective mGluR5 antagonists provide highly effective treatments for pain.

These findings are based on experiments performed with a new class of compounds which display a high degree of selectivity and affinity as antagonists of the human and rat mGluR5 (selective mGluR5 antagonists). Selective mGluR5 antagonists, as used herein, typically exhibit about 100 fold greater activity at an mGluR5 receptor than at an mGluR1 receptor, preferably about 200 fold greater activity and most preferably about 400 fold greater activity.These selective mGluR antagonists are 2-arylalkenyl-, 2-heteroarylalkenyl-, 2-arylalkynyl-, 2-heteroaryl-alkynyl-, 2-arylazo- and 2-heteroarylazo- pyridines, more particularly 6-methyl-2-(phenylazo)-3-pyridinol, (E)-2-methyl-6-styryl-pyridine and compounds of formula I 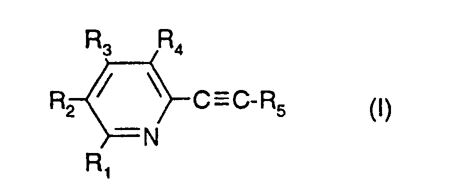 wherein
- R₁: is hydrogen, (C₁₋₄) alkyl, (C₁₋₄)alkoxy, cyano, ethynyl or di(C₁₋₄)alkylamino,
- R₂: is hydrogen, hydroxy, carboxy, (C₁₋₄) alkoxycarbonyl, di(C₁₋₄)alkylaminomethyl, 4-(4-fluoro-benzoyl)-piperidin-1-yl-carboxy, 4-t.-butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy,
- R₃: is hydrogen, (C₁₋₄) alkyl, carboxy, (C₁₋₄)alkoxycarbonyl, (C₁₋₄)alkylcarbamoyl, hydroxy(C₁₋₄)alkyl, di(C₁₋₄)alkylaminomethyl, morpholinocarbonyl or 4-(4-fluorobenzoyl)-piperidin-1-yl-carboxy,
- R₄: is hydrogen, hydroxy, carboxy, (C₂₋₅)alkanoyloxy, (C₁₋₄)alkoxycarbonyl, amino (C₁₋₄)alkoxy, di(C₁₋₄)alkylamino(C₁₋₄)alkoxy, di(C₁₋₄)alkylamino(C₁₋₄)alkyl or hydroxy(C₁₋₄)alkyl, and
- R₅: is a group of formula 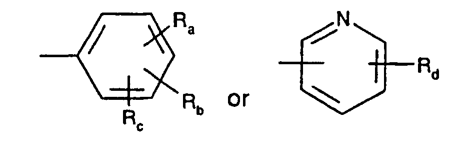
wherein
- Rₐ and R_{b}: independently are hydrogen, halogen, nitro, cyano, (C₁₋₄)alkyl, (C₁₋₄)alkoxy, trifluoromethyl, trifluoromethoxy or (C₂₋₅)alkynyl, and
- R_{c}: is hydrogen, fluorine, chlorine bromine, hydroxy (C₁₋₄)alkyl, (C₂₋₅)alkanoyloxy, (C₁₋₄)alkoxy or cyano, and
- R_{d}: is hydrogen, halogen or (C₁₋₄)alkyl,
in free form or in form of pharmaceutically acceptable salts.

More particularly the findings are based on experiments performed with compounds including 2-[2-(pyridin-3-yl)ethenyl]-6-methyl-pyridine, 3-methoxy-6-methyl-2-m-tolylethynyl-pyridine, 2-methyl-6-(2,3,5-trichloro-phenylethynyl)-pyridine, 2-methyl-6-(phenylethynyl)-pyridine and 2-(3-fluoro-phenylethynyl)-6-methyl pyridine (used as free bases).

The compounds of formula I can be prepared by reacting a compound of formula II 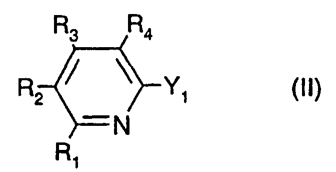 with a compound of formula III

Y₂ - R₅ (III)

in which one of Y₁ and Y₂ denotes a reactive esterified hydroxy group or a halogen such as bromine or iodine and the other one represents a group Y₃-C≡C- in which Y₃ is hydrogen or a metallic group, and R₁, R₂, R₃, R₄ and R₅ are as defined above and functional groups R₁, R₂, R₃ and R₄ as well as functional substituents of R₅ may be temporarily protected.

The reaction can be performed according to known methods, e.g. Heck and Sonogashira coupling or Grignard. The starting materials are known or can be obtained from known materials using conventional methods.

It has been found that the compounds of formula I are useful as modulators of mGluRs, particularly as selective mGluR5 antagonists.

Modulation of mGluRs can be demonstrated in a variety of ways, inter alia, in binding assays and functional assays such as second messenger assays or measurement of changes in intracellular calcium concentrations. For example, measurement of the inositol phosphate turnover in recombinant cell lines expressing hmGluR5a showed, for the compounds of formula I, IC₅₀ values of about 1 nM to about 50µM.

In particular, the compounds of formula I exhibit a marked and selective modulating, especially antagonistic, action at human mGluRs, especially mGluR5. This can be determined in vitro for example at recombinant human metabotropic glutamate receptors, especially PLC-coupled subtypes thereof such as mGluR5, using different procedures like, for example, measurement of the inhibition of the agonist induced elevation of intracellular Ca²⁺ concentration in accordance with L. P. Daggett et al. Neuropharm. Vol. 34, pages 871-886 (1995), P. J. Flor et al., J. Neurochem. Vol. 67, pages 58-63 (1996) or by determination to what extent the agonist induced elevation of the inositol phosphate turnover is inhibited as described by T. Knoepfel et al. Eur. J. Pharmacol. Vol. 288, pages 389-392 (1994), L. P. Daggett et al., Neuropharm. Vol. 67, pages 58-63 (1996) references cited therein. Isolation and expression of human mGluR subtypes are described in US-Patent No. 5,521,297. The compounds showed IC₅₀ vatues for the inhibition of the quisqualate-induced inositol phosphate turnover, measured in recombinant cells expressing hmGluR5a, of about 1 nM to about 50 µM.

Activity of mGluR5 antagonists as analgesics according to the invention can be demonstrated in models of persistent inflammatory pain and of neuropathic pain, performed as described below:

Oral administration of selective mGluR5 antagonists, for example as defined above, dose-dependently reverses mechanical hyperalgesia in the complete Freund's adjuvant rat model of inflammatory pain (Bartho et al., Naunyn Schmiedebergs Arch. Pharmacol. 342, 666-670, 1990). In this model, oral administration of antagonists of formula I, for example, produces a maximal reversal of between 60-95% reversal of inflammatory hyperalgesia with ED₅₀'s ranging between 4 and 25 mg/kg. The anti-hyperalgesic effects are of good duration (greater than 5 hours) and the onset is very rapid.

These results indicate that selective mGluR5 antagonists are useful in inflammatory pain.

Intraplantar administration of specific mGluR5 antagonists, for example as defined above, dose-dependently reverses mechanical hyperalgesia in the mouse partial sciatic nerve ligation model of neuropathic pain (according to a modification of Seltzer et al., Pain 43: 205-218, 1990). In this model, intraplantar administration of antagonists of formula I, for example, produces a significant reversal of mechanical hyperalgesia at doses of about 1 to about 100 mg/kg.

These findings indicate that the use for treating pain according to the invention is not limited to the treatment of inflammatory pain.

Analgesic effect achieved according to the invention is therefore suitable for the treatment of pain of various genesis or aetiology, in particular in the treatment of inflammatory pain and associated hyperalgesia, neuropathic pain and associated hyperalgesia, chronic pain, e.g. severe chronic pain, post-operative pain and pain associated with various conditions including cancer, angina, renal or billiary colic, menstruation, migraine and gout.

Inflammatory pain may be of diverse genesis, including arthritis and rheumatoid disease, teno-synovitis and vasculitis. Neuropathic pain includes trigeminal or herpetic neuralgia, diabetic neuropathy pain, causalgia, low back pain and deafferentation syndromes such as brachial plexus avulsion.

Activity of mGluR5 antagonists in anxiety according to the invention can be demonstrated in standard models such as the elevated plus maze in mice, the stress-induced hypothemia in mice and the social exploration test in rats, as described below:

In the elevated plus maze in OF1-mice [R.G. Lister, Psychopharmacology-Berl. 92, 180-185 (1987)], compounds of formula I, for example, increase the incidence of entries onto the open arms and the time spent on the open arms of the elevated plus maze on administration of doses of about 0.1 to about 100 mg/kg. The test may also be performed in male C57/BL6 mice, according to Razo et al. [Naunyn-Schmiedeberg's Arch. Pharmacol. 337, 675-678, 1988].

In the stress-induced hypothermia test in mice [B. Olivier et al., Euro. Neuropsychopharmacol. 4, 93-102 (1994)], compounds of formula I, for example, attenuate stress-induced hypothermia in OF1-mice at doses of about 1 to about 100 mg/kg.

In the social exploration test in rats, compounds of formula I, for example, increase the amount of social contact with the resident animal at doses of about 0.03 to 3 mg/kg. The test is performed as follows:

Male adult Sprague Dawley rats ("residents") and male young lister Hooded rats ("intruders") are used. "Intruders" are housed in pairs and "residents" are individually housed for two weeks before the test in plastic cages (Macrolon, 42 x 26 x 15 cm). All treatments are given to the "intruder" rats, only. The test-compound is administered orally (2 ml/kg). Two additional groups are included: controls receive 0.5% Methocel and an additional group is treated with the benzodiazepine chlordiazepoxide which serves as positive standard.
Twelve rats are used per group. Pairs consisting of one "intruder" rat and one "resident" rat are assigned at random to one of the experimental or the control groups. In each pair only the "intruder" is orally treated before being placed into the home age of a "resident" animal. The duration of active approach behaviours of the "intruder" rat (sniffing, anogenital exploration, nosing, grooming, licking, playing) towards the "resident" is manually registered and cumulatively recorded over a period of 5 minutes. All observations are made during the light phase (11 a.m. to 4 p.m.) in the home cage of the "resident".

In accordance with the above, the present invention provides:
a) the use of a mGluR5 antagonist to manufacture a medicament for the treatment of pain.

For the new uses according to the invention, the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 100 mg/kg body weight. In larger mammals, for example humans, an indicated daily dosage is in the range from about 5 to about 1000 mg of a compound for use according to the invention conveniently administered, for example, in divided doses up to five times a day.

Pharmaceutical compositions incorporating as active agent a mGluR5 antagonist are administered alone or in combination with pharmaceutically acceptable carriers, in either single or multiple doses. Suitable pharmaceutical carriers include inert solid diluents or fillers, sterile aqueous solutions, and various nontoxic organic solvents. The pharmaceutical compositions formed by combining the mGluR5 antagonist with the pharmaceutically acceptable carrier are then readily administered. These pharmaceutical carriers can, if desired, contain additional ingredients such as flavorings, binders, excipients, and the like.

Further embodiments of the invention provide articles of manufature containing package inserts with instructions for therapeutic use, packaging material and a formulation of one or more of the mGluR5 antagonist containing pharmaceutical compositions. The instructions for use will commonly identify administering the mGluR5 antagonist to ameliorate one or more symptoms of a dysfunction having a pain and/or anxiety component. The article of manufacture will also commonly contain a label indicating the compound, or composition, and its use for ameliorating one or more symptoms associated with the subject dysfunction.

The method of treating pain is intended to mean a method of delivering to a subject in need thereof a pharmaceutical preparation of mGluR5 antagonist with the aim of treating or preventing one or more symptoms of a dysfunction having a pain and/or anxiety component. The subject method includes delivering the preparation to a patient i) before the dysfunction has been diagnosed, e.g., prophylactic protocols delivered with the aim of preventing development of the dysfunction, as well as, ii) after the dysfunction has been diagnosed, e.g., therapeutic protocols.

In accordance with said method for treating pain and anxiety the mGluR5 antagonist is introduced in the structure of any medicinal form or composition. It is used as a solitary agent of medication or in combination with other medicinal preparations. Since the pharmacokinetics and pharmacodynamics of the mGluR 5 antagonist will vary in different patients, the most preferred method for achieving a therapeutic concentration in a tissue is to gradully escalate the dosage and monitor the clinical effects. The initial dose, for such an escalating dosage regimen of therapy, will depend upon the route of administration.

In addition to the finding of the remarkable activity of selective mGluR5 antagonists in the treatment of pain, it has surprisingly been found that hyperalgesic effects of mGluR antagonists are primarily mediated by peripherally expressed mGluRs, particularly mGluR5. This finding is totally unexpected in view of available evidence based on the following studies:

Electrophysiological studies of mGluRs have demonstrated that their activation strongly contributes to synaptic modulation in the central nervous system (CNS). Pharmacological and physiological studies of the spinal cord reflex suggest that mGluRs can both attenuate or enhance the motor output of the spinal cord (see Boxall et al., Neuroscience, 82:591-602, 1998). Intracellular studies have revealed that membrane properties of wide dynamic range interneurons and ventral horn neurons of the spinal cord are also directly affected by mGluR activation (Morisset and Nagy, J. Neurophysiol. 76:2794-2798, 1996; Liu and King, Br. J. Pharmacology. 116,105P, 1995).

Molecular biological studies have confirmed the expression of RNA's for mGluRs in mammalian CNS. Receptor proteins have also been described in mammalian brain for mGluR1-5, mGluR7 and mGluR8 sub-types. These receptor subtypes appear to be localised on neurons both pre- and post-synaptically, and also appear in glial cells. The presence of mGluR mRNA in the adult rat spinal cord has been demonstrated using in situ hybridisation techniques (Boxall et al. 1998 - see above). mGluRs 1, 3-5 and 7 subtype mRNA's are expressed in the rat spinal cord. Furthermore, immunohistochemistry techniques have demonstrated the expression of mGluR5 protein in the human and rat spinal cord and in the rat dorsal root ganglion cells (Valerio et al., Neuroscience Research. 28:49-57, 1997).

In vivo electrophysiological studies have revealed that spinal cord mGluR activation contributes to the development of spinal hyperexcitability (see Boxall et al. 1998 for review).

Behavioural pharmacological studies in rats indicate that the intrathecally administered mGluR group I agonist 3,5-dihydroxyphenylglycine (DHPG) induced an increase in spontaneous nociceptive responses in the rat (Fisher and Coderre, Neuroreport. 9:1169-1172, 1998). Further evidence for a spinal role of mGluR Group I receptors in nociceptive processing was indicated by the report of antinociceptive effects of intrathecally administered anti-rat mGluR1 and mGluR5 antibodies. Both of these antibodies reversed the spontaneous nociceptive responses evoked by intrathecal administration of DHPG (Fundytus et al., Neuroreport. 9:731-735, 1998). In addition, they both reversed the cold allodynia that developed following sciatic nerve injury in the rat, indicating that spinal mGluR1 and mGluR5 receptors may play a role in neuropathic pain.

All the available evidence based on the above-mentioned studies indicates that mGluR involvement in nociceptive processing is restricted to the CNS. Therefore, for analgesic efficacy, it would have been expected that therapy with mGluR antagonists would require access to the CNS, e.g. central administration or ability of the antagonist to pass the blood-brain barrier.

This finding according to the invention that the hyperalgesic effects of mGluR antagonists are primarily mediated by peripherally expressed mGluRs, particularly mGluR5, can be demonstrated in the standard models described below:

On intracerebroventricular or intrathecal administration of mGluR antagonists capable of penetrating the blood-brain barrier, e.g. specific mGluR5 antagonists in the complete Freund's adjuvant rat model (Bartho et al., 1990 as above), the mGluR antagonists produce only weak anti-hyperalgesic effects.

The brain and spinal cord sites are therefore unlikely to be the primary sites of action following oral administration.

In a naïve rat hind paw test of mechanical hyperalgesia (Randall and Selitto, Arch. Int. Pharmacodyn. Ther. 111:409-419, 1957), the following rank order of potency is obtained for glutamate receptor agonists: glutamate - 2-chloro-3-hydroxyphenylglycine (CHPG) > DHPG > NMDA > AMPA > (±)-2-aminobicyclo[3.1.0]hexane-2,6-dicarboxylic acid (LY 314582) > L-4-phosphono-2-amino-butiric acid (L-AP4). Of the receptor selective compounds tested, those acting at Group I mGluRs were the most potent hyperalgesic agents.

These results demonstrate that the mGluR Group I receptors are particulary involved in nociceptive transmission and that they are expressed peripherally.

On co-administration of the mGluR Group I agonist DHPG to the rat hind paw in the same model according to Randal and Selitto, the mGluR5 antagonists dose-dependently inhibit the DHPG-induced hyperalgesia, while the mGluR Group I antagonist (S)-4-carboxyphenylglycine [(S)-4C-PG], which is selective for mGluR1 over mGluR5 receptors, has limited effect.

These results indicate that the mGluR5 receptor is particularly involved in nociceptive transmission and confirm that it is expressed peripherally.

The above findings indicate that hyperalgesia associated to inflammatory pain can be treated with mGluR antagonists, e.g. mGluR antagonists having a mGluR5 antagonistic component. Moreover they indicate that a mGluR antagonist which does not (or is administered in such a way that it does not) substantially act at central mGluR receptors, while being substantially free of central effects, will not be less active as to its anti-hyperalgesic activity than a mGluR antagonist which penetrates the CNS.

In accordance with the above, the present invention also provides:
a) The use of a mGluR antagonist in the manufacture of a pharmaceutical composition for the treatment of pain by interaction of said antagonist primarily or predominantly at peripheral mGluR receptors.

Preferably said analgesic effect is achieved exclusively or substantially exclusively at peripheral mGluR receptors.

Predominant interaction at peripheral mGluR receptors is preferably achieved by chosing an active agent which does not substantially penetrate the CNS or is administered in such a way that it does not substantially penetrate the CNS.

Modes of administration which are such that the administered mGluR antagonist does not substantially penetrate the CNS include transdermal administration.

For transdermal administration, the mGluR antagonist may be administered in any conventional liquid or solid transdermal pharmaceutical composition, e.g. as described in Remington's Pharmaceutical Sciences 16th Edition Mack; Sucker, Fuchs and Spieser, Pharmazeutische Technologie 1st Edition, Springer and in GB 2098865 A or DOS 3212053, the contents of which are incorporated herein by reference.

Appropriate dosages of the mGluR antagonist for said analgesic effect are as described above for the use of mGluR5 antagonists in pain and anxiety.

Preferred mGluR5 antagonists for use according to the invention include compounds of formula I as defined above, in free form or in form of pharmaceutically acceptable salts. Representative compounds of formula I include 2-methyl-6-(phenylethynyl)-pryridine, 2-[(pyridine-3-yl)ethynyl]-6-methyl-pyridine, 2-(3-fluoro-pheny(ethynyl)-6-methyl-pyridine and (3-{2-[2-trans-(3,5-dichloro-phenyl)-vinyl]-6-methyl-pyridine-3-yloxy}-propyl)-dimethyl-amine. These and further compounds, and groups of compounds, of formula I for use according to the invention, as well as their preparation, are disclosed for example in WO 99/02497, incorporated herein by reference.

The tolerability of the mGluR5 antagonists of formula I may be determined in conventional manner. At the doses administered in the above indicated tests, no substantial toxicological effect is detected. Also in standard mutagenicity assays, e.g. the Ames screen, the compounds do not show evidence of a mutagenic potential.

## Claims

1. The use of a mGluR antagonist in the manufacture of a pharmaceutical composition for transdermal administration, for the treatment of pain.

## Patentansprüche

1. Verwendung eines mGluR-Antagonisten bei der Herstellung einer pharmazeutischen Zusammensetzung für transdermale Verabreichung zur Behandlung von Schmerz.

## Revendications

1. Utilisation d'un antagoniste du mGluR dans la fabrication d'une composition pharmaceutique pour l'administration transdermique, pour le traitement de la douleur.
